# EUROPEAN PATENT SPECIFICATION

(11) **EP 2 053 969 B1**
(45) Date of publication and mention of the grant of the patent: **30.05.2012**
(21) Application number: 07825994.2
(22) Date of filing: 07.08.2007
(51) Int. Cl.: A61B 5/11

(54) **A BED WITH INTEGRATED SENSOR UNIT FOR A PATIENT**
BETT MIT INTEGRIERTER SENSOREINHEIT FÜR EINEN PATIENTEN
LIT AVEC UNITÉ DE CAPTEUR INTÉGRÉE POUR UN PATIENT

(30) Priority: 14.08.2006 EP 06118867
(43) Date of publication of application: 06.05.2009
(73) Proprietor: Philips Intellectual Property & Standards GmbH, 20099 Hamburg (DE); Koninklijke Philips Electronics N.V., 5621 BA Eindhoven (NL)
(72) Inventor: BRAUERS, Andreas, NL-5656 AE Eindhoven (NL); DORSCHEID, Ralf, NL-5656 AE Eindhoven (NL); JOHNEN, Frank, NL-5656 AE Eindhoven (NL)
(74) Representative: Schouten, Marcus Maria
(86) International application number: PCT/IB2007/053108
(87) International publication number: WO 2008/020363

(56) References cited:
- EP-A- 0 488 552
- EP-A- 1 792 596
- US-A1- 2003 090 383
- US-A1- 2006 028 350

## Description

The present invention relates to a bed with integrated sensor unit for a patient. Furthermore the invention relates to a method of providing a bed with a sensor unit for a patient.

Monitoring of patients is a standard tool in different situations, mostly in hospital settings. Depending on the diagnosis, different parameters are monitored and different monitoring techniques are known, according to which sensor units are integrated into a hospital bed or the like. Parameters that are needed often are weight, heart rate, breathing rate and breathing abnormalities, as well as special movement patterns of the patient, e.g. during sleep. It requires a significant technical effort and different techniques to measure these quantities. Especially monitoring the weight is quite expensive. For those patients, for whom weight measurements are not required, a small and inexpensive design would be more attractive. It would also be attractive to equip a bed with such a measuring apparatus on demand, i.e. only if required.

US 2003/090383 describes a patient movement detection system for a bed including a load cell mounting assembly and US 2006/0028350 describes an apparatus and method for monitoring a patient in a hospital bed.

It is an object of the present invention to provide a simple and reliable technique for monitoring patients in a bed.

The object of the present invention is achieved by a bed for a patient as defined in independent claim 1, comprising a bedframe with at least a first and a second bedframe section, said bedframe sections being connected to each other by means of at least one hinge, the bed further comprising a sensor unit, characterized in that the sensor unit is connected with the pivot of the hinge, and is adapted to measure the movement of a bedframe section in a direction substantially perpendicular to the rotary axis of the pivot.

This object is achieved according to the invention by a method of providing a bed for a patient with a sensor unit as defined in independent claims 7 or 8, said bed comprising a bedframe with with at least a first and a second bedframe section, said bedframe sections being connected to each other by means of at least one hinge, the method comprising the step of replacing the original pivot of the hinge with a substitutional pivot, to which a sensor unit is connected or connecting to the original pivot of the hinge a pivot extension, to which a sensor unit is connected, said sensor unit being adapted to measure a movement of one of the bedframe sections in a direction substantially perpendicular to the rotary axis of the pivot.

The present invention describes a design of a bed and a sensor unit. Furthermore the present invention describes a method of handling said sensor unit with said bed. A bed according to the present invention is defined as a surface or any other device to rest on or to sit on etc., e.g. a conventional bed, a hospital bed, a care bed, a couch, a conventional chair, a dentist's chair, a wheelchair, an (operating) table, etc. However, the present invention is preferably applicable in hospital and nursing home settings. Accordingly the bed is preferably a hospital bed or a care bed.

A core idea of the invention is to integrate sensor units in the hinges of the bed's bedframe, in particular on the hinges which are used to connected bedframe sections to each other. Preferably, the sensor units are integrated in the hinges of the supporting surface of the bed, since in this way the most valuable information about patient movements can be obtained in a very simple and reliable way. The integration is achieved according to the invention in a way that allows a miniaturization of the overall sensing equipment. Furthermore an on demand retrofit of the sensor unit is possible. Thus not every bed has to provide these integrated sensors units, but can be equipped with the sensor units when relevant. For example, using the present invention a normal hospital bed can easily be retrofit to obtain an intensive care bed. The retrofit can be done by care personal, no technician is required. Additionally, the suggested integration of the sensor units does not restrict the overall design of the bed in terms of flexibility and moving parts. Furthermore, this solution can be realized at low cost.

The present invention is not limited to a certain type of sensor element. The sensor element of the sensor unit can be for example a force sensitive, pressure sensitive, or acceleration sensitive sensor element, etc. The sensor element may work based on capacitive measurements, piezoelectric measurements, seismic measurements, gyroscopic measurement, etc. The sensor element can also be a hydraulic element connected to a pressure sensor or the like.

These and other aspects of the invention will be described in detail hereinafter, by way of example, with reference to the following embodiment and the accompanying drawings; in which:
Fig.1 shows a schematic illustration of three bedframe sections of a hospital bed and the position of a sensor unit,
Fig. 2 shows a schematic illustration (cross-sectional view) of a pivot hinge with a sensor unit according to a first embodiment of the invention in a first position,
Fig. 3 shows a schematic illustration (cross-sectional view) of a pivot hinge with a sensor unit according to a first embodiment of the invention in a second position,
Fig. 4 shows a schematic illustration (cross-sectional view) of a pivot hinge with a sensor unit according to a second embodiment of the invention in a first position, and
Fig. 5 shows a schematic illustration (cross-sectional view) of a pivot hinge with a sensor unit according to a second embodiment of the invention in a second position.

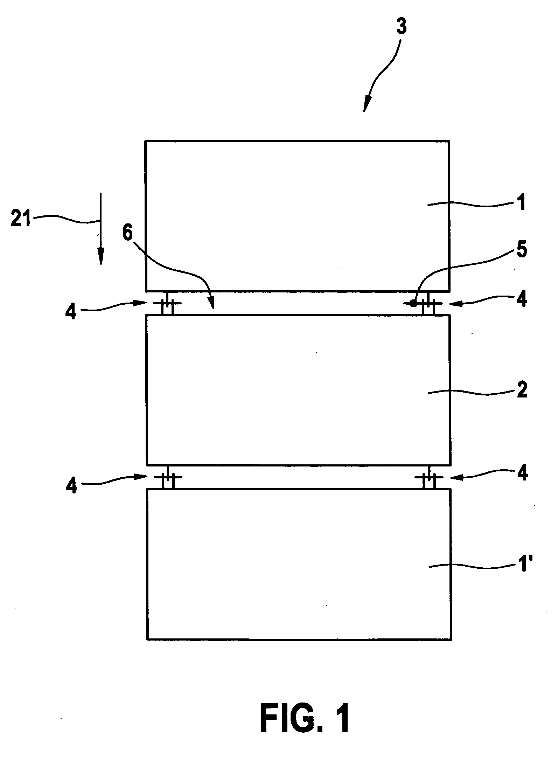

Fig. 1 illustrates a first outer bedframe section 1, a middle bedframe 2 section, and a second outer bedframe section 1' of a typical hospital bedframe 3 of a hospital bed. The bedframe sections 1, 2 are configurable in terms of height of the patient and positioning of different areas of the bedstead relative to each other. This is achieved by means of hydraulics and mechanically (not shown) assisted by hinges with rigid pivots. A number of hinges 4 connecting the bedframe sections 1, 2 are shown. One hinge 4 is provided with a sensor unit 5 according to the present invention. The sensor unit 5 is positioned in the gap 6 between the two bedframe sections 1, 2. This position of the sensor unit 5 allows for the measurement of forces and relative movements of the bedframe sections 1, 2 on which a patient (not shown) is lying. Such measurement enables an analysing unit (not shown) connected to the sensor unit 5 to analyze the measuring signals of the sensor unit 5 in order to determine the cardiopulmonary performance and/or the activity of a patient lying on the hospital bed. The analysing unit is preferably connected to the sensor unit 5 by means of a wireless communication line such that there are no cables or the like necessary, which could interfere with the handling of the hospital bed. In particular the analyzing unit is adapted to characterize the patient's movements and extract heart rate, breathing rate etc. and to monitor these data over a period of time. Design and operation of such an analyzing unit is well known in the art.

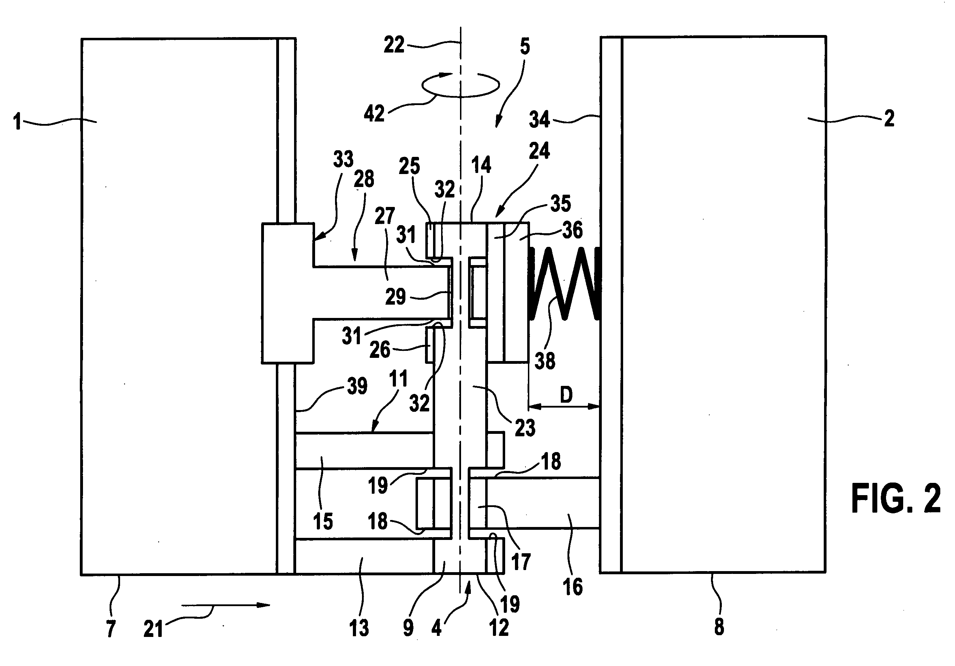

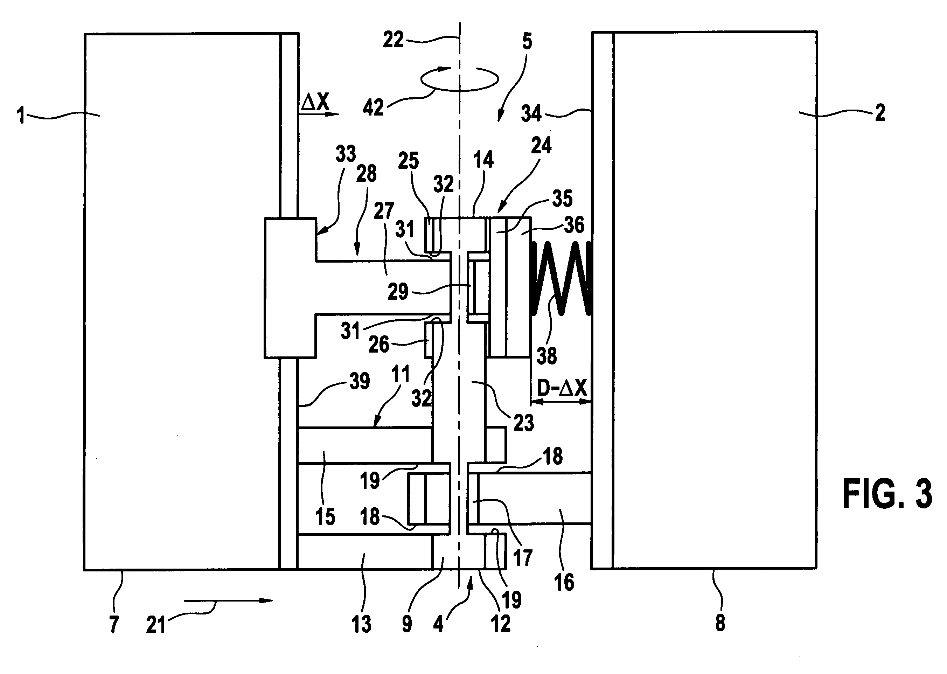

Design and position of a sensor unit 5 according to a first embodiment of the invention are illustrated in Figs. 2 and 3. The first bedframe section 1 and the second bedframe section 2 are connected to each other near their outer edges 7, 8 using the hinge 4. The pivot 9 of the hinge 4 is rigidly coupled to an U-shaped connector 11 of the first bedframe section 1. One end 12 of the pivot 9 ends with the outer leg 13 of the U-shaped connector 11, whereas the other end 14 of the pivot 9 extends beyond the inner leg 15 of the U-shaped connector 11. In-between the two legs 13, 15 of the U-shaped connector 11 a connector arm 16 of the second bedframe section 2 is positioned. The connector arm 16 comprises a bore hole 17, which receives the pivot 9. The side surfaces 18 of the connector arm 16 are guided by the inner surfaces 19 of the two legs 13, 15 of the U-shaped connector 11.

The bore hole 17 provides a slackness for the pivot 9, i.e. the diameter of the bore hole 17 is larger than the diameter of the pivot 9. This slackness does not only provide a space for rotating of the pivot 9 in the bore hole 17. Additionally the slackness is extended in a way that it allows the pivot 9 to move in a moving direction 21 perpendicular to the rotary axis 22 of the pivot 9 without forming a closed linkage (force closure) with the second bedframe section 2, if the movement of the first bedframe section 1 does not exceed a limit value. In other words, the first bedframe section 1 can move perpendicular to the rotary axis 22 of the pivot 9, i.e. horizontally with respect to the operating position of the hospital bed, without being in direct contact with the second bedframe section 2.

On the portion 23 of the pivot 9, which extends beyond the inner leg 15 of the U-shaped connector 11, an U-shaped support 24 is rigidly fixed. The U-shaped support 24 is spaced well apart from the outer hinge 4. The other end 14 of the pivot 9 ends with the outer leg 25 of the U-shaped support 24. In-between the outer leg 25 and the inner leg 26 of the U-shaped support 24 an arm 27 of a support member 28 is positioned. The arm 27 of the support member 28 comprises a bore hole 29, which receives a part of the extended portion 23 of the pivot 9. The side surfaces 31 of the arm 27 of the support member 28 are guided by the inner surfaces 32 of the two legs 25, 26 of the U-shaped support 24. To the arm 27 of the support member 28 a clamping device 33 is connected. The clamping device 33 is adapted to connect the support member 28 to the front side 39 of the first bedframe section 1.

The bore hole 29 provides a slackness for the pivot 9, i.e. a space for rotating of the pivot 9 in the bore hole 29. The bore hole 29, which preferably is provided with an oil film or the like, is adapted such that the pivot 9 can also perform very small movements in a direction perpendicular to the rotary axis 22 of the pivot 9. However, the slackness of the bore hole 29 is significant smaller than the extended slackness of the bore hole 17 of the connector arm 11.

To the base element 35 of the U-shaped support 24 the sensor unit 5 is connected. The sensor unit 5 comprises a force sensitive sensor element 36. Between the sensor element 36 and the front side 34 of the second bedframe section 2 a resilient element in form of a spring 38 is provided. The spring 38 is in working contact with the sensor element 36 on the one hand and rests against the front side 34 of the second bedframe section 2 on the other hand. Thus, the spring 38 ensures a permanent contact of the sensor element 36 with the second bedframe section 2. This permanent contact is supported by an adjustment tool, e.g. in form of an adjusting ring (not shown), which is located between the sensor unit 5 and the base element 35 of the U-shaped support 24. By means of the adjusting ring an initial tension is provided to the spring 38. Alternatively the initial tension of the spring 38 results solely from the spring characteristics and no adjusting ring is used. Because of the initial tension of the spring 38, the slackness of the bore hole 29 is factual abrogated, i.e. the U-shaped support 24 is in direct contact with the pivot 9. Instead of a spring, another resilient element can be used, e.g. a rubber disk.

With the outer hinge 4 as described above the two bedframe sections 1, 2 can rotate with respect to each other in rotating direction 42 around the rotary axis 22 of the pivot 9. The sensor unit 5 and/or the spring 38 is adapted in a way, that in all rotary positions the sensor element 36 of the sensor unit 5 is in permanent pretensioned contact with the corresponding front side 34 of the second bedframe section 2. For example the spring can obtain the form of a spherical shaped hood, which is imposed on the sensor element 36 and which rests against the front side 34 of the second bedframe section 2 in a way that ensures a permanent contact of the sensor element 36 with said second bedframe section 2 in all rotary positions.

The sensor unit 5 is connected to the first bedframe section 1 via the support member 28 and the pivot 9 of the hinge 4 is in a fixed rigid position. With this an exactly defined measuring position of the sensor unit 5 is guaranteed. Since the sensor unit 5 is rigidly coupled to the pivot 9 and the pivot 9 is guided in the bore hole 29 of the arm 27 of the support member 28, which is rigidly fixed to the first bedframe section 1, a movement ΔX of the first bedframe section 1 in a moving direction 20 substantially perpendicular to the rotary axis 22 of the pivot 9 leads to a corresponding movement ΔX of the extended portion 23 of the pivot 9 and thus to a corresponding movement ΔX of the sensor unit 5 relative to the second bedframe section 2, see Fig. 3. The distance D between the sensor element 36 and the front side 34 of the second bedframe section 2 decreases to D-ΔX. This movement is sensed by the sensor element 36 and subsequently used for analyzing the patient's movement, heart rate etc. In other words, a (horizontal) force, induced by a patient lying on the hospital bed, is redirected from the first bedframe section 1 via the pivot 9 to the inner hinge, which is formed by the extended portion 23 of the pivot 9 in the bore hole 29 of the support member 28, without a direct contact to the second bedframe section 2 in the outer hinge 4. The slackness of the bore hole 17 in the connector arm 16 preferably exceeds the maximum measuring movement of the sensor unit 5 relative to the second bedframe section 2. The slackness of the bore hole 29 in the support member 28 preferably exceeds twice the maximum horizontal movement ΔXₘₐₓ of the first bedframe section 1.

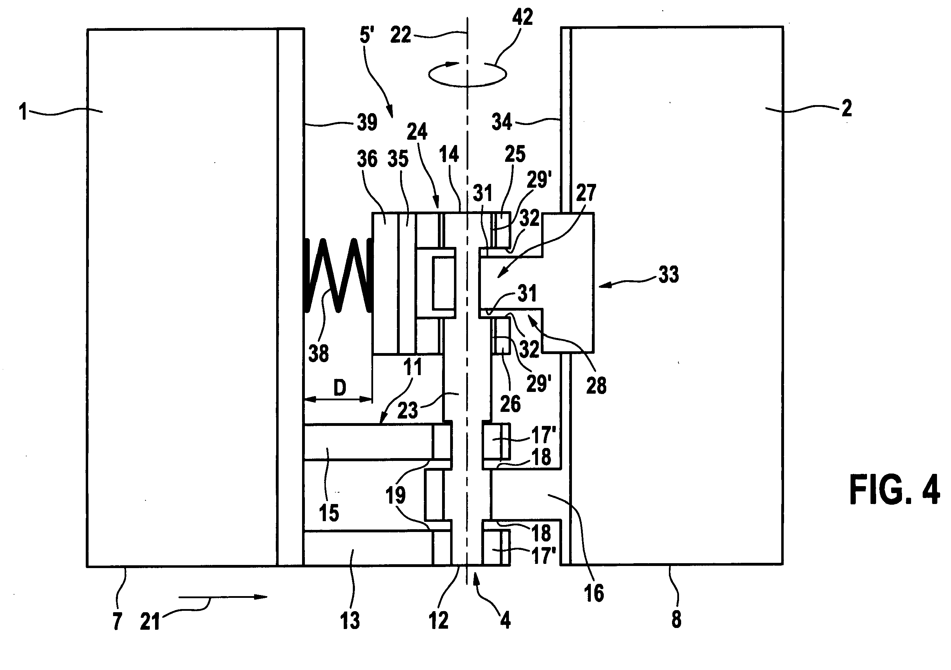

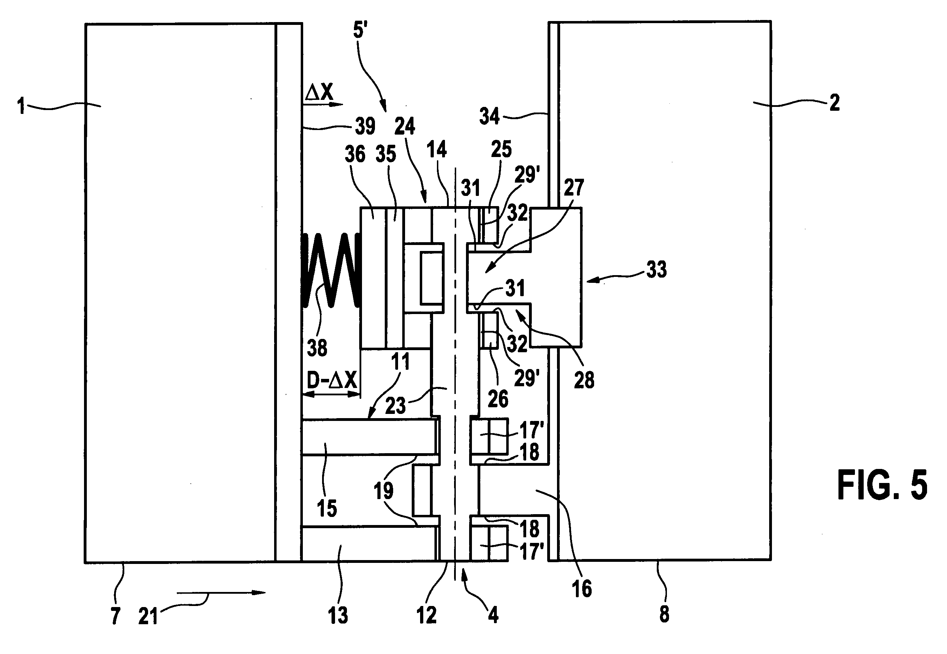

Design and position of a sensor unit 5' according to a second embodiment of the invention are illustrated in Figs. 4 and 5. The first bedframe section 1 and the second bedframe section 2 are connected to each other near their outer edges 7, 8 using the hinge 4. The first bedframe section comprises an U-shaped connector 11. Each of the two legs of the connector 11 comprises a bore hole 17' receiving the pivot 9. One end 12 of the pivot 9 ends with the outer leg 13 of the U-shaped connector 11, whereas the other end 14 of the pivot 9 extends beyond the inner leg 15 of the U-shaped connector 11. In-between the two legs 13, 15 of the U-shaped connector 11 a connector arm 16 of the second bedframe section 2 is positioned. The pivot 9 of the hinge 4 is rigidly coupled to the connector arm 16. The side surfaces 18 of the connector arm 16 are guided by the inner surfaces 19 of the two legs 13, 15 of the U-shaped connector 11.

The bore holes 17' provide a slackness for the pivot 9, i.e. the diameter of the bore holes 17' are larger than the diameter of the pivot 9. This slackness does not only provide a space for rotating of the pivot 9 in the bore holes 17'. Additionally the slackness is extended in a way that it allows the pivot 9 to move in a moving direction 21 perpendicular to the rotary axis 22 of the pivot 9 without forming a closed linkage (force closure) with the first bedframe section 1, if the movement of the first bedframe section 1 does not exceed a limit value. In other words, the first bedframe section 1 can move perpendicular to the rotary axis 22 of the pivot 9, i.e. horizontally with respect to the operating position of the hospital bed, without being in direct contact with the second bedframe section 2.

An U-shaped support 24, which is spaced well apart from the outer hinge 4, comprises two legs 25, 26. Each leg comprises a bore hole 29', which receives a part of the extended portion 23 of the pivot 9, which extends beyond the inner leg 15 of the U-shaped connector 11. The other end 14 of the pivot 9 ends with the outer leg 25 of the U-shaped support 24. In-between the outer leg 25 and the inner leg 26 of the U-shaped support 24 an arm 27 of a support member 28 is positioned. The arm 27 of the support member 2 is rigidly fixed to the pivot 9. The side surfaces 31 of the arm 27 of the support member 28 are guided by the inner surfaces 32 of the two legs 25, 26 of the U-shaped support 24. To the arm 27 of the support member 28 a clamping device 33 is connected. The clamping device 33 is adapted to connect the support member 28 to the front side 34 of the second bedframe section 2.

The bore holes 29' provide a slackness for the pivot 9, i.e. a space for rotating of the pivot 9 in the bore holes 29'. The bore holes 29', which preferably are provided with an oil film or the like, is adapted such that the pivot 9 can also perform very small movements in a direction perpendicular to the rotary axis 22 of the pivot 9. However, the slackness of the bore holes 29' is significant smaller than the extended slackness of the bore hole 17' of the legs 13, 15 of the connector 11.

To the base element 35 of the U-shaped support 24 the sensor unit 5 is connected. The sensor unit 5 comprises a force sensitive sensor element 36. Between the sensor element 36 and the front side 39 of the first bedframe section 1 a resilient element in form of a spring 38 is provided. The spring 38 is in working contact with the sensor element 36 on the one hand and rests against the front side 39 of the first bedframe section 1 on the other hand. Thus, the spring 38 ensures a permanent contact of the sensor element 36 with the first bedframe section 1. This permanent contact is supported by an adjustment tool, e.g. in form of an adjusting ring (not shown), which is located between the sensor unit 5 and the base element 35 of the U-shaped support 24. By means of the adjusting ring an initial tension is provided to the spring 38. Alternatively the initial tension of the spring 38 results solely from the spring characteristics and no adjusting ring is used. Because of the initial tension of the spring 38, the slackness of the bore holes 29 are factual abrogated, i.e. the U-shaped support 24 is in direct contact with the pivot 9.

With the outer hinge 4 as described above the two bedframe sections 1, 2 can rotate with respect to each other in rotating direction 42 around the rotary axis 22 of the pivot 9. In all rotary positions the sensor element 36 of the sensor unit 5 is in permanent pretensioned contact with the corresponding front side 39 of the first bedframe section 1. The sensor unit 5 is connected to the second bedframe section 2 via the support member 28 and the pivot 9 of the hinge 4 is in a fixed rigid position. With this an exactly defined measuring position of the sensor unit 5 is guaranteed.

Since the slackness of the bore holes 29' is abrogated by the spring 38, a movement ΔX of the first bedframe section 1 in a moving direction 20 substantially perpendicular to the rotary axis 22 of the pivot 9 leads to a decrease of the distance D between the sensor element 36 and the front side 39 of the first bedframe section 1 to D-ΔX, see Fig. 5. This movement is sensed by the sensor element 36 and subsequently used for analyzing the patient's movement, heart rate etc.

In other words, a (horizontal) force, induced by a patient lying on the hospital bed, is measured at the inner hinge, which is formed by the extended portion 23 of the pivot 9 in the bore holes 29' of the legs 25, 26, without a direct contact to the second bedframe section 2 in the outer hinge 4. The slackness of the bore holes 17' in the legs 13, 15 of the connector 11 preferably exceeds the maximum measuring movement of the sensor unit 5 relative to the second bedframe section 2. The slackness of the bore holes 29' in the legs 25, 26 preferably exceeds twice the maximum horizontal movement ΔXₘₐₓ of the first bedframe section 1.

A standard hospital bed can easily be retrofit to obtain an intensive care bed by providing one or more sensor units 5, 5' to the hinges 4 between the bedframe sections 1, 2, 1' of the bed. Preferably all hinges 4 of the hospital bed will be provided with a sensor unit 5. 5' as described. For obtaining analysable results it is however adequate to provide a single sensor unit 5. 5' to one hinge 4. This can be done either by replacing the original pivot of the hinge 4 with a substitutional pivot 9, to which a sensor unit 5, 5' is already connected. In Figs. 2 to 5 such an substitutional pivot 9 is illustrated. Alternatively, this can be achieved by connecting to the original pivot of the hinge 4 a pivot extension (not shown), to which a sensor unit 5, 5' is connected. Such pivot extension would preferably comprise a fastener to ensure a secure connection between the original pivot and the pivot extension, e.g. in form of a clamp or the like. In other words, the placement or replacement of the sensor unit 5, 5' can be realized while using a standard coupling on the pivot or while replacing the whole pivot.

The placement procedure of the sensor unit 5, 5' comprises the following steps: First the original pivot is replaced by a substitutional pivot 9 or a pivot extension is connected. Subsequently a mechanical fixture, here in form of the support member 28, is connected with the bedframe section, which is not in permanent contact with the sensor element 5, 5'. Furthermore, the sensor unit 5, 5' is adjusted to the opposite bedframe section. Finally a cable connection or a wireless communication link (not shown) is established between the sensor unit 5, 5' and the analyzing unit.

The analyzing unit is adapted to analyze the plurality of recorded signals according to a predefined analyzing pattern. For this purpose the analysing unit preferably comprises a processing unit which is adapted for performing all tasks of calculating and computing the measured data as well as determining and assessing results. This is preferably achieved by means of a computer software comprising computer instructions adapted for carrying out said analyzing steps, when the software is executed in the processing unit. The processing unit itself may comprise functional modules or units, which are implemented in form of hardware, software or in form of a combination of both.

### REFERENCE NUMERALS

- 1: outer bedframe section
- 2: middle bedframe section
- 3: bedframe
- 4: hinge
- 5: sensor unit
- 6: gap
- 7: outer edge
- 8: outer edge
- 9: pivot
- 10: (free)
- 11: connector
- 12: pivot end
- 13: outer leg
- 14: pivot end
- 15: inner leg
- 16: connector arm
- 17: bore hole
- 18: side surface
- 19: inner surface
- 20: (free)
- 21: moving direction
- 22: rotary axis
- 23: extended portion
- 24: support
- 25: outer leg
- 26: inner leg
- 27: arm
- 28: support member
- 29: bore hole
- 30: (free)
- 31: side surface
- 32: inner surface
- 33: clamping device
- 34: front side
- 35: base element
- 36: sensor element
- 37: (free)
- 38: spring
- 39: front side
- 40: (free)
- 41: (free)
- 42: rotating direction

## Claims

1. A bed for a patient, comprising a bedframe (3) with at least a first and a second bedframe section (1, 2) for a patient to lie on, said bedframe sections (1, 2) being connected to each other by means of at least one hinge (4), the bed further comprising a sensor unit (5, 5') and an analyzing unit for providing monitoring of activity and/or cardiopulmonary performance of a patient lying on the bed based on signals from the sensor unit (5, 5'), **characterized in that** the sensor unit (5, 5')
- is connected with the pivot (9) of the hinge (4), and
- is adapted to measure the movement of one of said bedframe sections (1, 2) relative to the other bedframe section (1, 2) in a horizontal direction (21) substantially perpendicular to the rotary axis (22) of the pivot (9).

2. The bed as claimed in claim 1, **characterized in that** one of the bedframe sections (1, 2) is rigidly coupled to the pivot (9) and the other bedframe section (2, 1) comprises a guiding element (17, 17') for receiving the pivot (9) and allowing the pivot (9) to rotate within the guiding element (17, 17').

3. The bed as claimed in claim 2, **characterized in that** the guiding element (17, 17') having a slackness that allows the pivot (9) to move perpendicular to the rotary axis (22) of the pivot (9) without forming a closed linkage with the other bedframe section (2, 1), if the movement of the one bedframe section (1, 2) does not exceed a limit value.

4. The bed as claimed in claim 1, **characterized in that** the sensor unit (5, 5') is rigidly coupled to the pivot (9) and a resilient element (38) is used to ensure a permanent contact of the sensor element (36) of the sensor unit (5, 5') with one of the bedframe sections (1, 2).

5. The bed as claimed in claim 1, **characterized in that** the pivot (9) is extended beyond the hinge (4) and the sensor element (36) is coupled to a portion (23) of the pivot (9), which is spaced apart from the hinge (4).

6. The bed as claimed in claim 5, **characterized in that** a guiding element (29, 29') is provided for receiving at least partly said portion (23) of the pivot (9) and allowing the pivot (9) to rotate within the guiding element (29, 29'), said guiding element (29, 29') is connected with a bedframe section (1, 2), which is not in permanent contact with the sensor element (36).

7. A method of providing a bed for a patient with a sensor unit (5, 5'), said bed comprising a bedframe (3) with at least a first and a second bedframe section (1, 2) for a patient to lie on, said bedframe sections (1, 2) being connected to each other by means of at least one hinge (4), the method comprising the step of replacing the original pivot of the hinge (4) with a substitutional pivot (9), to which a sensor unit (5, 5') is connected, said sensor unit (5) being adapted to measure a movement of one of the bedframe sections (1, 2) relative to the other bedframe section (1, 2) in a horizontal direction (21) substantially perpendicular to the rotary axis (22) of the pivot (9), the method further comprising providing an analyzing unit for providing monitoring of activity and/or cardiopulmonary performance of a patient lying on the bed based on signals from the sensor unit (5, 5').

8. A method of providing a bed for a patient with a sensor unit (5, 5'), said bed comprising a bedframe (3) with at least a first and a second bedframe section (1, 2) for a patient to lie on, said bedframe sections (1, 2) being connected to each other by means of at least one hinge (4), the method comprising the step of connecting to the original pivot of the hinge (4) a pivot extension, to which a sensor unit (5, 5') is connected, said sensor unit (5) being adapted to measure a movement of one of the bedframe sections (1, 2) relative to the other bedframe section (1, 2) in a horizontal direction (21) substantially perpendicular to the rotary axis (22) of the pivot (9), the method further comprising providing an analyzing unit for providing monitoring of activity and/or cardiopulmonary performance of a patient lying on the bed based on signals from the sensor unit (5, 5').

## Patentansprüche

1. Bett für einen Patienten mit einem Bettrahmen (3) mit zumindest einem ersten und einem zweiten Bettrahmenabschnitt (1, 2), auf dem ein Patient liegen kann, wobei die genannten Bettrahmenabschnitte (1, 2) anhand mindestens eines Scharniers (4) miteinander verbunden sind, wobei das Bett ferner eine Sensoreinheit (5, 5') und ein Analysegerät zum Schaffen einer auf Signalen von der Sensoreinheit (5, 5') basierenden Überwachung der Aktivität und/oder der kardiopulmonalen Leistung eines auf dem Bett liegenden Patienten umfasst, **dadurch gekennzeichnet, dass** die Sensoreinheit (5, 5')
- mit dem Drehzapfen (9) des Scharniers (4) verbunden ist, und
- so ausgelegt ist, dass es die Bewegung eines der genannten Bettrahmenabschnitte (1, 2) hinsichtlich des anderen Bettrahmenabschnitts (1, 2) in einer horizontalen Richtung (21), die im Wesentlichen senkrecht zur Drehachse (22) des Zapfens (9) steht, misst.

2. Bett nach Anspruch 1, **dadurch gekennzeichnet, dass** einer der Bettrahmenabschnitte (1, 2) starr mit dem Zapfen (9) verbunden ist und der andere Bettrahmenabschnitt (2, 1) ein Führungselement (17, 17') zur Aufnahme des Zapfens (9) umfasst, das es dem Zapfen (9) gestattet, sich im Führungselement (17, 17') zu drehen.

3. Bett nach Anspruch 2, **dadurch gekennzeichnet, dass** das Führungselement (17, 17') ein Spiel hat, dass es dem Zapfen (9) gestattet, sich senkrecht zur Drehachse (22) des Zapfens (9) zu bewegen, ohne eine geschlossene Verbindung mit dem anderen Bettrahmenabschnitt (2, 1) zu bilden, wenn die Bewegung des einen Bettrahmenabschnitts (1, 2) nicht einen Grenzwert überschreitet.

4. Bett nach Anspruch 1, **dadurch gekennzeichnet, dass** die Sensoreinheit (5, 5') starr mit dem Zapfen (9) verbunden ist und ein elastisches Element (38) dazu verwendet wird, einen dauerhaften Kontakt zwischen dem Sensorelement (36) der Sensoreinheit (5, 5') und einem der Bettrahmenabschnitte (1, 2) sicherzustellen.

5. Bett nach Anspruch 1, **dadurch gekennzeichnet, dass** der Zapfen (9) über das Scharnier (4) hinausragt und das Sensorelement (36) mit einem Teil (23) des Zapfens (9) verbunden ist, der von dem Scharnier (4) räumlich getrennt ist.

6. Bett nach Anspruch 5, **dadurch gekennzeichnet, dass** ein Führungselement (29, 29') zur zumindest teilweisen Aufnahme des genannten Teils (23) des Zapfens (9) geschaffen wird, das es dem Zapfen (9) gestattet, sich in dem Führungselement (29, 29') zu drehen, wobei das genannte Führungselement (29, 29') mit einem Bettrahmenabschnitt (1, 2) verbunden ist, der keinen dauerhaften Kontakt mit dem Sensorelement (36) hat.

7. Verfahren zum Schaffen eines Bettes für einen Patienten mit einer Sensoreinheit (5, 5'), wobei das genannte Bett einen Bettrahmen (3) mit zumindest einem ersten und einem zweiten Bettrahmenabschnitt (1, 2) umfasst, auf dem ein Patient liegen kann, wobei die genannten Bettrahmenabschnitte (1, 2) anhand mindestens eines Scharniers (4) miteinander verbunden sind, wobei das Verfahren den Schritt des Ersetzens des ursprünglichen Zapfens des Scharniers (4) durch einen Ersatzzapfen (9) umfasst, mit dem eine Sensoreinheit (5, 5') verbunden ist, wobei die genannte Sensoreinheit (5) so ausgelegt ist, dass sie die Bewegung eines der Bettrahmenabschnitte (1, 2) hinsichtlich des anderen Bettrahmenabschnitts (1, 2) in einer horizontalen Richtung (21), die im Wesentlichen senkrecht zur Drehachse (22) des Zapfens (9) steht, misst, wobei das Verfahren ferner die Schaffung eines Analysegerätes zum Schaffen einer auf Signalen von der Sensoreinheit (5, 5') basierenden Überwachung der Aktivität und/oder der kardiopulmonalen Leistung eines auf dem Bett liegenden Patienten umfasst.

8. Verfahren zum Schaffen eines Bettes für einen Patienten mit einer Sensoreinheit (5, 5'), wobei das genannte Bett einen Bettrahmen (3) mit zumindest einem ersten und einem zweiten Bettrahmenabschnitt (1, 2) umfasst, auf dem ein Patient liegen kann, wobei die genannten Bettrahmenabschnitte (1, 2) anhand mindestens eines Scharniers (4) miteinander verbunden sind, wobei das Verfahren den Schritt des Anbringens einer Zapfenverlängerung an dem ursprünglichen Zapfen des Scharniers (4) umfasst, mit der eine Sensoreinheit (5, 5') verbunden wird, wobei die genannte Sensoreinheit (5) so ausgelegt ist, dass sie die Bewegung eines der genannten Bettrahmenabschnitte (1, 2) hinsichtlich des anderen Bettrahmenabschnitts (1, 2) in einer horizontalen Richtung (21), die im Wesentlichen senkrecht zur Drehachse (22) des Zapfens (9) steht, misst, wobei das Verfahren ferner die Schaffung eines Analysegerätes zum Schaffen einer auf Signalen von der Sensoreinheit (5, 5') basierenden Überwachung der Aktivität und/oder der kardiopulmonalen Leistung eines auf dem Bett liegenden Patienten umfasst.

## Revendications

1. Lit pour un patient, comprenant un châlit (3) avec au moins une première et une seconde section de châlit (1, 2) pour étendre un patient, lesdites sections de châlit (1, 2) étant raccordées l'une à l'autre au moyen d'au moins une charnière (4), le lit comprenant en outre une unité de capteur (5, 5') et une unité d'analyse permettant de conférer une surveillance de l'activité et/ou des performances cardiopulmonaires d'un patient étendu sur le lit d'après des signaux provenant de l'unité de capteur (5, 5'), **caractérisé en ce que** l'unité de capteur (5, 5')
- est raccordée au pivot (9) de la charnière (4), et
- est adaptée pour mesurer le mouvement d'une desdites sections de châlit (1, 2) par rapport à l'autre section de châlit (1, 2) dans une direction horizontale (21) sensiblement perpendiculaire à l'axe de rotation (22) du pivot (9).

2. Lit selon la revendication 1, **caractérisé en ce qu'**une des sections de châlit (1, 2) est couplée de façon rigide au pivot (9) et l'autre section de châlit (2, 1) comprend un élément de guidage (17, 17') pour recevoir le pivot (9) et permettre au pivot (9) de tourner autour de l'élément de guidage (17, 17').

3. Lit selon la revendication 2, **caractérisé en ce que** l'élément de guidage (17, 17') a un jeu qui permet au pivot (9) de se déplacer de façon perpendiculaire à l'axe de rotation (22) du pivot (9) sans former une tringlerie serrée avec l'autre section de châlit (2, 1) si le mouvement d'une des sections de châlit (1, 2) ne dépasse pas une valeur limite.

4. Lit selon la revendication 1, **caractérisé en ce que** l'unité de capteur (5, 5') est couplée de façon rigide au pivot (9) et un élément résilient (38) est utilisé pour garantir un contact permanent de l'élément de capteur (36) de l'unité de capteur (5, 5') avec une des sections de châlit (1, 2).

5. Lit selon la revendication 1, **caractérisé en ce que** le pivot (9) est étendu au-delà de la charnière (4) et l'élément de capteur (36) est couplé à une portion (23) du pivot (9), qui est espacé de la charnière (4).

6. Lit selon la revendication 5, **caractérisé en ce qu'**un élément de guidage (29, 29') est disposé pour recevoir au moins partiellement ladite portion (23) du pivot (9) et permettre au pivot (9) de tourner au sein de l'élément de guidage (29, 29'), ledit élément de guidage (29, 29') est raccordé à une section de châlit (1, 2), qui n'est pas en contact permanent avec l'élément de capteur (36).

7. Procédé permettant de doter un lit pour un patient d'une unité de capteur (5, 5'), ledit lit comprenant un châlit (3) avec au moins une première et une seconde section de châlit (1, 2) pour étendre un patient, lesdites sections de châlit (1, 2) étant raccordées l'une à l'autre au moyen d'au moins une charnière (4), le procédé comprenant l'étape consistant à remplacer le pivot original de la charnière (4) par un pivot de substitution (9), auquel l'unité de capteur (5, 5') est raccordée, ladite unité de capteur (5) étant adaptée pour mesurer un mouvement de l'une des sections de châlit (1, 2) par rapport à l'autre section de châlit (1, 2) dans une direction horizontale (21) sensiblement perpendiculaire à l'axe de rotation (22) du pivot (9), le procédé comprenant en outre la fourniture d'une unité d'analyse permettant de conférer une surveillance de l'activité et/ou des performances cardiopulmonaires d'un patient étendu sur le lit d'après des signaux provenant de l'unité de capteur (5, 5').

8. Procédé permettant de doter un lit pour un patient d'une unité de capteur (5, 5'), ledit lit comprenant un châlit (3) avec au moins une première et une seconde section de châlit (1, 2) pour étendre un patient, lesdites sections de châlit (1, 2) étant raccordées l'une à l'autre au moyen d'au moins une charnière (4), le procédé comprenant l'étape consistant à raccorder au pivot original de la charnière (4) une extension de pivot, à laquelle l'unité de capteur (5, 5') est raccordée, ladite unité de capteur (5) étant adaptée pour mesurer un mouvement de l'une des sections de châlit (1, 2) par rapport à l'autre section de châlit (1, 2) dans une direction horizontale (21) sensiblement perpendiculaire à l'axe de rotation (22) du pivot (9), le procédé comprenant en outre la fourniture d'une unité d'analyse permettant de conférer une surveillance de l'activité et/ou des performances cardiopulmonaires d'un patient étendu sur le lit d'après des signaux provenant de l'unité de capteur (5, 5').
